(19) Europäisches Patentamt
European Patent Office
Office européen des brevets

(11) Publication number: **0 289 118**
**A1**

(12) # EUROPEAN PATENT APPLICATION

(21) Application number: 88301885.5

(22) Date of filing: 03.03.88

(51) Int. Cl.[4]: C07D 498/08 , A61K 31/395 , A61K 31/65 , //(C07D498/08, 267:00,307:00)

The title of the invention has been amended (Guidelines for Examination in the EPO, A-III, 7.3).

(30) Priority: 03.03.87 ZA 871527

(43) Date of publication of application: 02.11.88 Bulletin 88/44

(84) Designated Contracting States: AT BE CH DE ES FR GB GR IT LI LU NL SE

(71) Applicant: SOUTH AFRICAN INVENTIONS DEVELOPMENT CORPORATION
Administration Building Council for Scientific and Industrial Research Scientia
Meiringnaude Road
Pretoria Transvaal(ZA)

(72) Inventor: Oberem, Peter Thomas
Plot 11
Mont Lorraine Transvaal(ZA)

(74) Representative: Ellis-Jones, Patrick George Armine et al
J.A. KEMP & CO. 14 South Square Gray's Inn
London WC1R 5EU(GB)

(54) **Antibiotic treatment of ectoparasite-borne diseases.**

(57) The use of a rifamycin antibiotic, particularly rifampicin, in the treatment of ectoparasite borne diseases in animals, particularly heartwater. The rifamycin antibiotic may be administered intravenously, intramuscularly, orally or topically.

EP 0 289 118 A1

# TREATMENT OF ECTOPARASITE BORNE DISEASES

## BACKGROUND OF THE INVENTION

This invention relates to the treatment of ectoparasite borne diseases, i.e. diseases transmitted by ectoparasites such as ticks and flies.

One of the most serious of all the ectoparasite borne diseases is *Cowdria ruminantium* infection, or heartwater as it is commonly known. Heartwater is an infectious, virulent, inoculable and non-contagious disease affecting domestic and wild ruminants. It is characterised in domestic ruminants by a high fever and acute gastroenteritis and hydropericardium, followed in acute and peracute forms by severe nervous symptoms. The causative agent is rickettsia, *Cowdria ruminantium* which is borne by ticks of the *Amblyomma* genus.

A variety of treatments of heartwater have been used and proposed. For example, formalin and arsenic have been administered, but with limited success. Various antibiotics have also been tried. Penicillin G has been found to be ineffective against heartwater *in vivo*, but has had some effect against *Cowdria ruminantium in vitro*.

Ampicillin has been found to be completely ineffective in clinical cases of heartwater. On the other hand, the tetracyclines as a group are effective for the specific treatment of heartwater. A successful treatment does, however, depend on many factors. These factors include timing, dose rate, formulation used, and route of administration. Of the various types of tetracyclines used, the most commonly used is oxytetracycline. However, it has been found that high daily dosages are required and in a recently published study it was observed that the persistence of fever was noticed despite two to three daily treatments at a dose rate of 10 mg/kg.

Thus, while there are various treatments available for heartwater, problems are associated with all of these treatments. The successful treatment of field cases of heartwater remains a problem not only in Southern Africa but in other African countries.

## SUMMARY OF THE INVENTION

It has been found that antibiotics of the rifamycin group are effective in the treatment of ectoparasite borne diseases. Thus, the invention provides, according to one aspect, the use of a rifamycin antibiotic in the treatment of ectoparasite borne diseases in animals.

The invention provides, according to another aspect, a composition for use in the treatment of ectoparasite borne diseases in animals which includes a rifamycin antibiotic in an effective amount and a suitable carrier.

## DETAILED DESCRIPTION OF THE INVENTION

Rifamycin antibiotics are known and used in the art and are described in the complete specification of South African Patent No. 65/3608. In particular, the antibiotics have the formula:

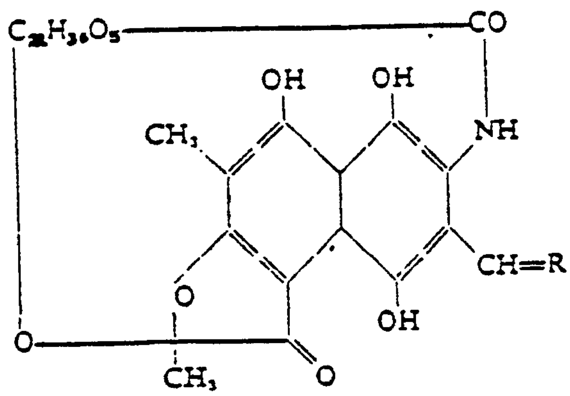

(I)

wherein R represents oxygen, H(OH), dialkoxy, imine, substituted imino, hydrazone and substituted hydrazono radicals.

The preferred rifamycin antibiotic is rifampicin which is a 3-(4-methyl-piperazinyl)-imino-methyl derivative of rifamycin SV and has the formula:

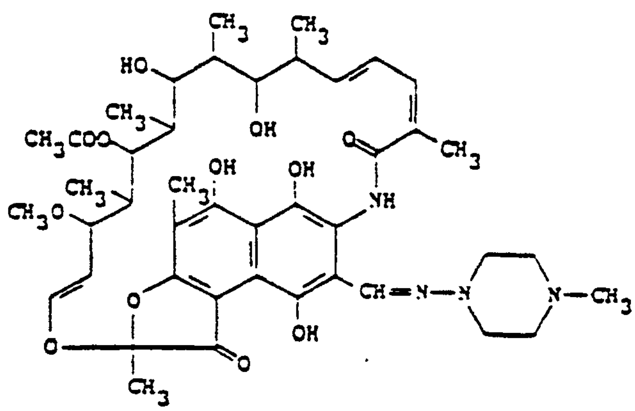

The rifamycin antibiotic may be administered to the animal intramuscularly, intravenously, orally (per os), or dermatologically (topically). For any particular administration, the formulation used will contain a sufficient quantity of the rifamycin antibiotic to be effective in the treatment of the disease and will contain known carriers and/or excipients.

The rifamycin antibiotic is preferably administered in a dose of 0,2 to 25 mg/kg of body weight of the animal. For intramuscular and intravenous administration the dosage will be at the lower end of this range. For oral and dermatological applications, the dosage will be at the higher end of the range.

It has been found that a combination of rifampicin and oxytetracycline is synergistic in the treatment of an ectoparasite borne disease, particularly Cowdria ruminantium infection in ruminants. This synergistic combination forms another aspect of the invention.

It has been found that the dermatological administration is particularly effective. An example of a suitable dermatological preparation is one containing a non-polar solvent capable of dissolving the rifamycin antibiotic, the rifamycin antibiotic in an effective quantity, and a suitable surface active agent. The function of the surface active agent is to allow the solvent to spread uniformly and effectively across the surface of the animal being treated. An example of a suitable surface active agent is xylene. Examples of suitable solvents are dimethyl sulfoxide (DMSO), dimethyl acetamide (DMA) and dimethyl formamide (DMF).

Examples of suitable compositions of the invention are set out below. Two of the compositions are topical, and two of the compositions are injectables.

| Topical 1 | Topical 2 | Injectable 1(im) | Injectable 2(im) |
|---|---|---|---|
| 200mg rifampicin | 1,0g rifampicin | 0,6g rifampicin | 4g rifampicin |
| 4ml DMSO | 15ml DMSO | 3,0ml DMSO | 14ml DMSO |
| 2ml Peanut oil | 5ml Xylene | | 1ml Benzyl alcohol |
| 2ml Xylene | | | 10ml Polyelthylene glycol |
| | | | 15ml Propylene glycol (USP) |
| Dosage | Dosage | Dosage | Dosage |
| 20mg/kg (0,02ml) | 20,0mg/kg | 10,0mg/kg | 10,0mg/kg |

Examples of ectoparasite borne diseases which can be treated with the use of rifamycin antibiotics are as follows:

(a) Cowdria ruminantium infection (heartwater) in ruminants,
(b) Erhlichia canis (tropical pancytopaenia) in dogs,
(c) Anaplasma marginale (gall sickness) in cattle,
(d) Babesia bigemina and Babesia bovis (redwater) in cattle,
(e) Babesia canis (biliary) in dogs,
(f) Babesia equi (biliary) in horses,
(g) Babesia felis (biliary) in cats.

The animals which are treated by the invention are in particular, domestic animals such as dogs, cats and horses and wild and domestic ruminants such as sheep, goat and cattle, and chickens.

Rifampicin has been subjected to several trials against a variety of diseases and infections in a variety of animals. Details of these trials are now provided.

Trial 1: Screening of various antimicrobials for efficacy against heartwater (Cowdria ruminantium infection) in mice.

Materials and Methods:

Groups of 10 six-to eight week-old Swiss white mice weighing 20 g to 25 g each were infected intraperitoneally (IP) with Cowdria ruminantium (Kümm strain) at a determined specific dosage as described by du Plessis and Kümm Journal of South African Veterinary Medical Association, 42, (1971) 217-221. On day 8 after infection the mice of each group were treated with a candidate remedy (see Table 1). On each occasion 1 group of 10 mice was included as an untreated control and another as a control group treated with oxytetracycline at 10 mg/kg. Where possible the recommended dosage and route of administration was employed. Thereafter the mice were monitored daily and the deaths recorded. Post mortem lesions were used to confirm heartwater as the cause of death. After deaths ceased, the percentage survival for each group was calculated. Any potentially effective antimicrobials were further processed.

All formulations except that of rifampicin for topical application are commercially available. The topical formulation of rifamycin was made up as follows:

200 mg rifamycin
4 ml dimethyl sulphoxide (DMSO)
2 ml peanut oil
2 ml xylene

Results:

Table 1: Results of an *in vivo* determination of the efficacy of various antimicrobials on *C. ruminantium*, Kümm strain, in mice

| Antimicrobial Remedy | Dose (mg/kg) | Route of Administration | No. of Mice Infected | % Survival (average incubation) |
|---|---|---|---|---|
| Controls (No Rx) | - | - | 30 | 10 (15.9 days) |
| Oxytetracyclines | 10 | IP | 50 | 82 (20) |
| Chloramphenicol | 50 | IP | 5 | 0 (14.5) |
| Sulphadimethyl pyrimidine + trimethoprim | 20 +4 | IP | 5 | 0 |
| Amikacin sulphate | 8 (b.i.d.) | IP | 3 | 33.3 |
| Trobamycin " | 1 (t.i.d.) | IP | 3 | 0 |
| Netilmycin " | 2 (t.i.d.) | IP | 3 | 0 |
| Neomycin " | 3 | IP | 5 | 0 |
| Gentamycin " | 4 (b.i.d.) | IP | 3 | 0 |
| Streptomycin | 4 (b.i.d.) | IP | 5 | 0 |
| Kanamycin | 10 | IP | 5 | 0 |
| Gloxazone | 2 | IP | 5 | 100 |
| Rifampicin | 20 | p.o. | 20 | 95 (27) |
| " | 10 | p.o. | 10 | 100 |
| " | 5 | p.o. | 20 | 95 |
| " | 2.5 | p.o. | 10 | 100 |
| " | 1.25 | p.o. | 10 | 30 |
| " | 20 (0.02 ml) | topical | 10 | 100 |

(1) p.o. = per os.

(2) Gloxazone, which is effective against *C. ruminantium*, is too toxic to be marketed.

(3) The topical formulation resulted in the mice showing some discomfort for about 10 minutes after application but no visible lesions.

Conclusions:

1. Nil % of the untreated controls survived while oxytetracycline treatment (positive controls) increased the survival rate of mice to 82%. Thus the mouse model can be regarded as a reliable test for the efficiacy of antimicrobials against C. ruminantium.

2. Treatment of C. ruminantium mice infected with rifampicin, both per os and topically, increases the survival rate of mice from 10% (untreated controls) to up to 100% depending on dosage.

3. None of the other antimicrobials tested was effective in the treatment of the infection.

Trial 2: In vivo determination of the efficacy of rifampicin treatment on C. ruminantium (Ball 3 strain) infection in sheep

Materials and Methods:

Fourteen heartwater-susceptible sheep were each inoculated intravenously with 5 ml heartwater-infected sheep blood (Onderstepoort Ball 3 heartwater vaccine). Their rectal temperatures were monitored daily and all the sheep except 2 controls were treated on the 2nd, 3rd or 4th day that their temperatures reached or exceeded 40°C. The 2 sheep treated per os were treated with a commercially available preparation as were the 2 sheep treated intravenously. The remedy used intramuscularly on 2 sheep was formulated as follows: 0.6 g rifampicin and 3 ml DMSO.

The pour-on formulation was made up as follows:

One gram rifamycin, 15 ml DMSO and 5 ml xylene. All surviving sheep were again challenged with 5 ml infected blood 3 weeks after recovery in order to ascertain the effect of rifampicin treatment on their immune status.

Results:

Table 2. The results of an in vivo determination of the efficacy of rifampicin treatment on Cowdria ruminantium (Ball 3 strain) in sheep.

| Dosage (mg/kg) | Route of Administration | No. of Sheep Infected | Survival (%) | Survival on Challenge (%) |
|---|---|---|---|---|
| - | - | 2 | 0 | - |
| 20 | i.v. | 2 | 100 | 100 |
| 20 | topical | 6 | 100** | 100 |
| 10 | i.m. | 2 | 100 | 100 |
| 20 | per os | 2 | 100* | 100 |

* Recovery was very slow.

None of the recovered sheep showed any clinical signs of heartwater as a result of the second challenge

** All 6 sheep showed moderate irritation for about 10 minutes after application. This disappeared and left no visible lesions.

Conclusion:

1. All 4 formulations were effective in the treatment of heartwater in sheep.
2. Sheep that recovered from heartwater infection after rifampicin treatment are immune to the disease.

Trial 3: Comparison of the minimum therapeutic dosage of rifampicin; oxytetracycline and a combination of these against Cowdria ruminantium (Kümm strain) in mice.

Materials and Methods:

15 Groups of Swiss white mice were infected with Kümm strain C. ruminantium as described in trial 1. On day 8 after infection all groups except the untreated controls, were treated with either rifampicin (per os) oxytetracyclines (intraperitoneally) or a combination of both at various dosage rates as indicated in table 3. Dilutions of both products were made using sterile water so that each mouse was treated with 0,25 ml. The percentage mortality was then determined for each group.

Results:

Table 3: Percentage survival of mice infected with C. ruminantium (Kümm strain) and treated with various dosages of oxytetracycline, rifampicin or combinations thereof.

| Remedy and Dosage (mg/kg) | | No. of Mice Infected | Survival (%) |
|---|---|---|---|
| Oxytetracycline | 1 | 40 | 62,5 |
| | 2 | 40 | 80 |
| | 4 | 20 | 75 |
| | 6 | 40 | 82,5 |
| | 8 | 40 | 85 |
| | 10 | 40 | 82,5 |
| Rifampicin | 0,1 | 40 | 52,5 |
| | 0,2 | 40 | 82,5 |
| | 0,4 | 40 | 92,5 |
| | 1 | 40 | 82,5 |
| | 2 | 40 | 60 |
| | 4 | 59 | 81,4 |
| Oxytetracycline<br>plus rifamycin | 2 mg/kg<br>0,2 mg/kg | 49 | 91,8 |
| Oxytetracycline<br>plus rifamycin | 1 mg/kg<br>0,1 mg/kg | 30 | 90,0 |

Conclusion:

1. 0,2 mg/kg rifamycin or 2 mg/kg oxytetracycline are the minimum effective dosages of each of these remedies.

2. Combinations of these appear to be at least as effective as the single products. Such combinations will probably be advantageous in delaying the emergence of resistant bacteria

Trial 4: To compare dimethylsulfoxide (DMSO) dimethylacetamide (DMA) dimethylformamide (DMF) as carriers in a pour-on rifampicin formulation for the treatment of heartwater (Cowdria ruminantium infection) in mice.

Materials and Methods:

The basic experimental design was as in trials 1 and 3. Forty mice were injected intraperitoneally with C. ruminantium (Kümm strain) inoculum. Ten remained untreated while 10 were treated once only on day 8 post-infection with one of each of the following pour-on formulations:

3 ml carrier : either DMSO; DMA or DMF
1 ml xylene
1 ml peanut oil
125 mg rifampicin

Each mouse was treated with 0,02 ml of this 25 mg/ml formulation (At 40 x 25 g mice this represents 0,8 ml/kg).

The percentage survival and average survival time were monitored and recorded as in trial 1. Any signs of irritation were also monitored.

Results:

Table 4: Percentage survival, and average survival time of and irritation shown by mice infected with C. ruminantium (Kümm strain) and treated with three pour-on rifampicin formulations.

| Formulation (Carrier) | Survival (%) | Average Survival time (days) | Irritation (immediate) |
|---|---|---|---|
| DMSO | 100 | - | Present |
| DMA | 100 | - | Absent |
| DMF | 100 | - | Absent |
| Controls (No R) | 20 | 16,2 | - |

1 week after treatment all mice treated showed a mild generalized dermatitis on the back. This had fully recovered by the end of the second week after treatment.

Conclusions:

1. All 3 substances appear to be equally effective as carriers in rifampicin pour-on formulations
2. DMSO formulation is more irritant to the skin of mice than DMA or DMF.

Trial 5: To confirm the efficacy of DMA and DMF pour-on rifampicin formulations in the treatment of heartwater (Cowdria ruminantium infection) in sheep

Materials and methods:

Fifteen heartwater-susceptible sheep were infected with C. ruminantium (Ball 3 strain) as described in trial 2.

On the 3rd or 4th day of pyrexia ( 40°C) 14 of the sheep were treated as indicated in table 5. The 15th sheep remained as an untreated control. Temperatures were monitored daily and mortalities noted: any signs of irritation caused by the remedies were noted.

Results:

Table 5: The efficacy of various pour-on rifampicin formulations in the treatment of heartwater in sheep

| FORMULATION | | DOSAGE | | DAY/S OF RX | SURVIVAL | IRRITATION |
|---|---|---|---|---|---|---|
| Carrier | Strength (mg/ml) | (mg/kg) | (ml/kg) | Post Infection | (Yes/No) | (Yes/No) |
| DMSO | 50 | 20 | 0,4 | 13 | Yes | Yes |
| | 50 | 20 | 0,4 | 12 | Yes | Yes |
| | 25 | 10 | 0,4 | 11 + 15 | Yes | Yes |
| | 25 | 10 | 0,4 | 12 + 17 | Yes | Yes |
| DMF | 50 | 20 | 0,4 | 12 | Yes | No |
| | 50 | 20 | 0,4 | 14 | Yes | No |
| | 25 | 10 | 0,4 | 11 | Yes | No |
| | 25 | 10 | 0,4 | 13 + 17 | Yes | No |
| DMA | 50 | 20 | 0,4 | 11 | Yes | No |
| | 50 | 20 | 0,4 | 11 | Yes | No |
| | 25 | 10 | 0,4 | 13 + 17 | Yes | No |
| | 25 | 10 | 0,4 | 13 + 17 | Yes | No |
| Oxytetracycline | 123 | 10 | 0,08* | 17 | Yes | No |
| Oxytetracycline (pos. contr) | 123 | 10 | 0,08* | 17 | No | No |
| Untreated control | - | - | - | - | No | No |

* Intravenous treatment

Conclusions:

1) DMF and DMA are at least as effective as DMSO
2) DMF and DMA do not cause irritation (which is caused by DMSO)
3) A minimum dosage between 10-and 20 mg/kg rifampicin is required in a pour-on formulation in order to safely apply one treatment only.

Trial 6: To determine whether a pour-on formulation as developed for rifampicin could be effectively used for oxytetracyclines

Materials and Methods:

An oxytetracycline formulation was prepared as follows:

250 mg oxytetracycline
3 ml DMF
1 ml xylene
1 ml sunflower oil

Twenty mice were infected with *C. ruminantium* (Kümm strain) as described in trial 1. On day 8 post-infection 15 of the mice were treated with 0,01 ml (20 mg/kg) dermally. Five mice were left as untreated controls.

Results:

Table 6: The efficacy of DMF containing oxytetracycline pour-on formulation in the treatment of mice infected with *C. ruminantium* (Kümm strain)

| Treatment | No. of Mice Treated | % Survival |
|---|---|---|
| Oxytetracycline pour-on | 15 | 0 |
| Untreated controls | 5 | 0 |

Conclusion:

The oxytetracycline pour-on formulation is ineffective.

Rifamycin Treatment of Anaplasmosis (*Anaplasma marginale* infection) in Cattle

*Anaplasma marginale* was first described by Theiler (1910) as the causative organism of anaplasmosis of cattle in South Africa. Anaplasmosis is an economically important, tick-borne, rickettsial disease of cattle in Africa, the Americas (North and South) and Australasia. Its control is based also on the chemotherapeutic use of oxytetracyclines and vector control.

Due to the expense of obtaining and perhaps losing cattle during a trial designed to establish the effect of rifamycins on anaplasmosis infection it was decide to use *Aegyptianella pullorum*, a parasite closely related to *Anaplasma*, in poultry as a model.

Trial 7: The determination of the effect of rifampicin treatment on the parasitaemia and haematocrit of chickens infected with Aegyptianella pullorium.

Materials and Methods

. Ten 4-week old broiler chickens were each inoculated intravenously with 0,5 ml A. pullorum infected blood. This blood freshly drawn in heparin and had a 6% parasitaemia. The haematocrit and parasitaemia of all the chickens was monitored daily thereafter. Once the average parasitaemia reached 5% (on day 10 after infection) 5 of the fowls were treated with rifampicin (commercially available oral formulation) at 20 mg/kg, i.e. 1 ml/kg of a 20 mg/ml solution. The remaining 5 chickens remained untreated as controls.

Results:

See Table 7.

Table 7 Average parasitaemia and haematocrit of the rifampicin treated and untreated control chickens infected with *Aegyptianella pullorum*

| | Day | 1 | 2 | 3 | 4 | 5 | 6 | 7 | 8 | 9 | 10* | 11 | 12 | 13 | 14 |
|---|---|---|---|---|---|---|---|---|---|---|---|---|---|---|---|
| Average | Treated | ,36 | - | - | ,46 | ,30 | ,26 | 1,82 | ,8 | 4,0 | 5,2 | 7,9 | 4,0 | 4,3 | 2,7 |
| Parasitaemia | Control | ,38 | - | - | ,38 | ,22 | ,28 | 4,1 | 1,8 | 2,6 | 5,9 | 11,1 | 19,1 | 24,8 | 28,2 |
| Average | Treated | 34,4 | - | - | 35,0 | 33,8 | 33,6 | 37,0 | 30,0 | 30,0 | 30,0 | 31,6 | 31,2 | 30,4 | 28,2 |
| Haematocrit | Control | 34,2 | - | - | 33,4 | 27,8 | 31,6 | 41,5** | 26,5 | 26,8 | 25,8 | 26 | 24,2 | 20,3 | 18,5 |

* Treatment with rifampicin

** Blood of 3 of these chickens clotted distorting this days average haematocrit for the control group

Conclusion:

Rifamipicin is an effective treatment for Aegyptianella pullorum infection of chickens. Rifampicin does not sterilize the infection. It could possibly thus prove effective against Anaplasma infection in cattle.

Trial 8: To determine the effect of rifampicin on Anaplasma marginale var. centrale infection in cattle.

Introduction:

In an attempt to isolate pure infections of A. marginale Theiler (1910) found an organism in a heifer from Aliwal North in the Cape Province of South Africa. This organism was smaller and more centrally placed in the erythrocyte and it caused less severe reactions in cattle than A. marginale. He called this organism Anaplasma marginale var. centrale (hereinafter called A. centrale) and also demonstrated that animals infected with A. centrale had sufficient immunity to protect them against A. marginale challenge.

As splenectomized bovines infected with A. centrale are used to produce vaccine in South Africa, it was decided to use them as a further model in the determination of the efficacy of the rifamycins against A. marginale.

Materials and Methods:

A splenectomized ox was infected with 8 ml of citrated A. centrale infected blood vaccine. The animal was thereafter monitored daily for packed cell volume (PCV), temperature and parasitaemia changes. Once the parasitaemia reached 4% the animal was bled (4,21) for vaccine and treated with 10 mg/kg rifampicin intramuscularly. The rifamycin was made up as follows:

4 g rifampicin; 14 ml DMSO; 1 ml benzyl alcohol; 10 ml polyethylene glycol 400 and 15 ml propylene glycol (USP). The bovine weighing 359 kg was given 36 ml of this in 4 injection sites of 9 ml each.

Results:

Table 8 The PCV (a measure of anaemia), parasitaemia and temperature during *A. centrale* infection of a splenectomized bovine both before and after treatment with rifampicin

| Date | PCV | Parasitaemia % | Temperature (°C) |
|---|---|---|---|
| 8/6/1987 | 30 | 0 | 37.5 |
| 26/6/87 | 30 | 0 | 38.0 |
| 1/7/87 | 30 | 0 | 38.5 |
| 2 | 30 | 1 | 38.6 |
| 3 | 30 | 4 | 39.0 |
| 4 | 25 | 7 | 38.4 |
| 5 | 28 | 12 | 39.6 |
| 6* | 22 | 19 | 38.8 |
| 7 | ND | 19 | 38.8 |
| 8 | ND | 23 | 38.4 |
| 9 | ND | 18 | 39.3 |
| 10 | 18 | 18 | 38.4 |
| 11 | 18 | 17 | 39.1 |
| 12 | 1y | 8 | 39.0 |
| 13 | 17 | 4 | 38.3 |

* Treatment with rifampicin, 10 mg/kg i.m.
ND = not done

Conclusion:

Rifampicin treatment is effective in controlling *A. centrale* infection in cattle.

Rifamycin treatment of biliary or babesiosis (*Babesia* spp. infection).

Trial 9: The effect of B. equi infection and rifamycin treatment on various blood parameters in the horse

Materials and Methods:

A 3½ month-old male boerperd foal was given 1 ml of B. equi infected blood intravenously on 2/2/87 at 11h40. Various criteria including: haemoglobin (Hb); red cell count (RCC); haematocrit (Ht) and parasitaemia were measured at suitable intervals thereafter. Once these former factors began declining and the parasitaemia had reached significant levels (3 plus) the foal was given 20 mg/kg rifampicin per os (9/2/87).

Results:

Table 9: The effect of B. equi infection and rifamycin treatment on various blood parameters in a 3½ month old foal

| Date | Hb (g/l) | RCC: (x10[illegible]/l) | Ht: (l/l) | Parasitaemia (0 - 1 - 3 plus) |
|---|---|---|---|---|
| 2/2/87 | 141 | 83,4 | 39 | 0 |
| 6/2/87 | 134 | 83,8 | 36 | 0 |
| 9/2/87 | 101 | 61,4 | 27 | 3+ |
| 10/2/87* | 83 | 50,0 | 22 | 3+ |
| 11/3/87 | 73 | 45,6 | 19 | 2+ |
| 12/2/87 | 81 | 5,17 | 22 | 0 |
| 16/2/87 | 94 | 5,83 | 26 | 0 |

* Treatment: 20 mg/kg rifampicin oral suspension per os.

The foal recovered completely.
The above data has been depicted on graph No. 5

Conclusions:

Rifampicin is effective in the treatment of Babesia equi.

**Claims**

1. A rifamycin antibiotic having the formula (I):

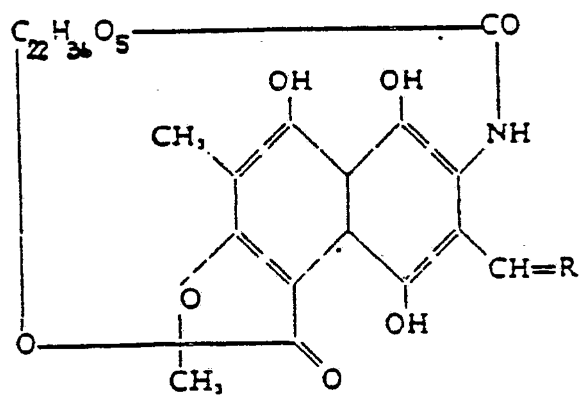

(I)

wherein R represents oxygen, H(OH), dialkoxy, imine, substituted imino, hydrazone and substituted hydrazono radicals,
for use in the treatment of ectoparasite borne diseases in animals.

2. The antibiotic according to claim 1 which is rifampicin.

3. The antibiotic according to claim 1 or claim 2 which is administered intravenously, intramuscularly, orally or dermatologically.

4. The antibiotic according to any one of the preceding claims which is administered in a dose of 0,2 to 25 mg/kg of body weight of the animal.

5. The antibiotic according to any one of the preceding claims wherein the animal to be treated is a domestic animal, a wild or domestic ruminant or a chicken.

6. The antibiotic according to any one of the preceding claims for use in the treatment of one or more of the following diseases in animals:

(a) Cowdria ruminantium infection (heartwater) in ruminants,
(b) Erhlichia canis (tropical pancytopaenia) in dogs,
(c) Anaplasma marginale (gall sickness) in cattle,
(d) Babesia bigemina and Babesia bovis (redwater) in cattle,
(e) Babesia canis (biliary) in dogs,
(f) Babesia equi (biliary) in horses,
(g) Babesia felis (biliary) in cats.

7. A composition for use in the treatment of ectoparasite borne diseases in animals comprising an effective quantity of a rifamycin antibiotic of the formula (I):

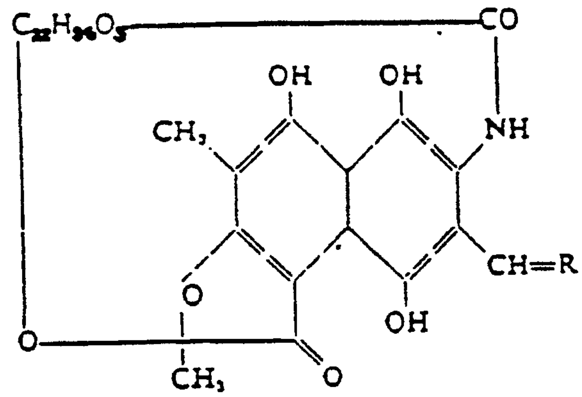

(I)

wherein R represents oxygen, H(OH), dialkoxy, imine, substituted imino, hydrazone and substituted hydrazono radicals,
and a suitable carrier.

8. A composition according to claim 7 for dermatological application wherein the carrier comprises a non-polar solvent capable of dissolving the antibiotic and a surface active agent.

9. A composition according to claim 8 wherein the non-polar solvent is selected from dimethyl sulfoxide, dimethyl acetamide and dimethyl formamide.

10. A composition according to claim 8 or claim 9 wherein the surface active agent is xylene.

11. A composition according to any one of claims 7 to 10 wherein the rifamycin antibiotic is rifampicin.

12. A composition according to any one of claims 7 to 11 which also comprises oxytetracycline in an effective amount.

13. The use of a rifamycin antibiotic of the general formula (I)

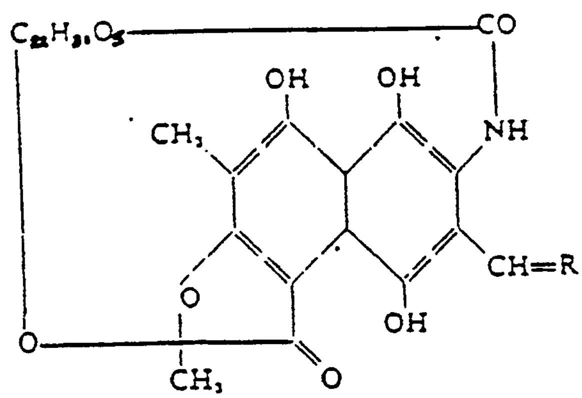

(I)

wherein R represents oxygen, H(OH), dialkoxy, imine, substituted imino, hydrazone and substituted hydrazono radicals,
for the manufacture of a composition for the treatment of ectoparasite borne diseases in animals.

14. The use according to claim 13 wherein the diseases are one or more of the following diseases:

(a) Cowdria ruminantium infection (heartwater) in ruminants,
(b) Erhlichia canis (tropical pancytopaenia) in dogs,
(c) Anaplasma marginale (gall sickness) in cattle,
(d) Babesia bigemina and Babesia bovis (redwater) in cattle,
(e) Babesia canis (biliary) in dogs,
(f) Babesia equi (biliary) in horses,
(g) Babesia felis (biliary) in cats.

15. The use according to claim 13 or claim 14 wherein the rifamycin antibiotic is rifampicin.

European Patent Office

# EUROPEAN SEARCH REPORT

Application number

EP 88301885.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| X | CHEMICAL ABSTRACTS, vol. 102, no. 13, April 1, 1985, Columbus, Ohio, USA<br>A.A. MC COLM et al.: "Evaluation of a range of antimicrobial agents against the parasitic protozoa, Plasmodium falciparum, Babesia rodhaini, and Theileria parva in vitro."<br>page 353, abstract no. 109 287z<br>& Ann. Trop. Med. Parasitol. 1984, 78(4), 345-54<br>-- | 1,6, 13,14 | C 07 D 498/08<br>A 61 K 31/395<br>A 61 K 31/65<br>//(C 07 D 498/08,<br>C 07 D 267:00,<br>C 07 D 307:00) |
| A | CHEMICAL ABSTRACTS, vol. 105, no. 13, September 29, 1986, Columbus, Ohio, USA<br>K.L. KUTTLER et al.: "Chemoprophylactic activity of imidocarb, diminazene and oxytetracyline against Babesia bovis and B. bigemina."<br>page 23, abstract no. 108 002c<br>& Vet. Parasitol. 1986, 21(2), 107-18<br>-- | 1,6,7, 12-14 | TECHNICAL FIELDS SEARCHED (Int. Cl 4)<br>C 07 D 498/00<br>A 61 K 31/00 |

The present search report has been drawn up for all claims

| Place of search | Date of completion of the search | Examiner |
|---|---|---|
| VIENNA | 06-06-1988 | JANISCH |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82

European Patent Office

# EUROPEAN SEARCH REPORT

Application number
EP 88301885.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| A | CHEMICAL ABSTRACTS, vol. 103, no. 21, November 25, 1985, Columbus, Ohio, USA<br>A.B. OGUNKOYA et al.: "Experimental and clinical trials of long acting oxytetracycline in the treatment of canine ehrlichiosis." page 22, abstract no. 171 488q<br>& Vet. Q. 1985, 7(2), 158-61<br>-- | 1,6,7, 12-14 | |
| A | CHEMICAL ABSTRACTS, vol. 96, no. 17, April 26, 1982, Columbus, Ohio, USA<br>J.L. DU PLESSIS: "The influence of of dithiosemicarbazone on the immunity of sheep to heartwater." page 35, abstract no. 135 400h<br>& Onderstepoort J. Vet. Res. 1981, 48(3), 175-6<br>-- | 1,6,13, 14 | |
| A | CHEMICAL ABSTRACTS, vol. 95, no. 9, August 31, 1981, Columbus, Ohio, USA<br>K.L. KUTTLER et al.: "Chemotherapy of babesiosis: a review." page 7, abstract no. 72 945w<br>& Proc. Int. Conf. Malar. Babesiosis, 1981, 65-85<br>-- | 1,6,12-14 | |

TECHNICAL FIELDS SEARCHED (Int Cl 4)

The present search report has been drawn up for all claims

| Place of search | Date of completion of the search | Examiner |
|---|---|---|
| VIENNA | 06-06-1988 | JANISCH |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82

European Patent Office

# EUROPEAN SEARCH REPORT

Application number EP 88301885.5

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl 4) |
| A | CHEMICAL ABSTRACTS, vol. 80, no. 15, April 15, 1974, Columbus, Ohio, USA<br>L.P. JOYNER: "Chemotherapy of anaplasmosis, babesiasis, and theileriasis."<br>page 3, abstract no. 78 258m<br>& Advan. Pharmacol. Chemother. 1973, 11, 321-55<br>---- | 1,6,12-14 | |
| | | | TECHNICAL FIELDS SEARCHED (Int Cl 4) |

The present search report has been drawn up for all claims

| Place of search | Date of completion of the search | Examiner |
|---|---|---|
| VIENNA | 06-06-1988 | JANISCH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document